# EUROPEAN PATENT SPECIFICATION

(11) **EP 1 114 074 B1**
(45) Date of publication and mention of the grant of the patent: **10.10.2007**
(21) Application number: 99943054.9
(22) Date of filing: 06.09.1999
(51) Int. Cl.: C08F 220/28, C08F 290/06, A61L 15/24, A61L 15/58, A61L 15/60

(54) **POLYMERIZABLE MATERIAL AND SHEET MATERIAL DERIVED THEREFROM**
POLYMERISIERBARES MATERIAL UND BLATTFÖRMIGES MATERIAL DARAUS
MATERIAU POLYMERISABLE ET MATERIAU EN FEUILLE DERIVE DUDIT MATERIAU

(30) Priority: 07.09.1998 GB 9819489
(43) Date of publication of application: 11.07.2001
(73) Proprietor: PolyBioMed Limited, Sheffield S9 3SP (GB)
(72) Inventor: AL-LAMEE, Kadem, Gayad, Yorkshire LS16 7AD (GB); TAKTAK, Yousef, Samih, Matlock,Derbyshire DE4 5LR (GB)
(74) Representative: Tollett, Ian
(86) International application number: PCT/GB1999/002772
(87) International publication number: WO 2000/014131

(56) References cited:
- EP-A2- 0 670 338
- WO-A1-99/34833
- US-A- 4 424 311
- US-A- 5 395 907
- US-A- 5 614 586
- US-A- 5 626 863
- US-A- 5 844 016
- Polymer preprint. (Am. Chem. Soc., Div. Polym. Chem.), Volume 34, No. 1, 1993, Hubbel, Jeffrey A. et al, "In vivo photopolymerization of PEG-based biodegradable hydrogels for the control of wound healing" pages 846 - 847, XP002900831
- Adv. Biomater. Biomed. Eng. Drug Delivery Syst., (Iketani conf. Biomed. Polym.), 5th (1996), Meeting Date 1995, Hubbell, Jeffrey A. et al: "Controll of healing with photopolymerizable biodegradable hydrogels", pages 179 - 182, XP002900832

## Description

### Field of the Invention

The present invention to a photopolymerizable composition, to an adhesive composition which is obtainable by curing said composition, and to the use of that composition, for example in the production of wound dressings, electrodes for application to the body of human beings or animals, or transdermal patches. The invention is particularly, but not exclusively, applicable to the production of sheet material for use as hydrogel wound dressings for the treatment of open wounds.

### Background to the Invention

### Skin-adherent materials for dressing wounds

Hydrogels based on carbohydrate-derived materials have been known for a number of years. For example PCT/WO95/17166 discloses a non-cytotoxic wound hydrating gel based on sodium/calcium alginate and sodium carboxymethylcelluose. Wound dressings based on systems of this kind were introduced in the 1980's and have the advantages that the hydrocolloids stimulated tissue growth, are easy to use, and provided a good barrier to microorganisms. Although they continue to be in use, they have a disadvantage that they are difficult to remove from the wound without damaging it,:and as they are opaque it is difficult to observe the progress of wound healing

A transparent wound dressing is available from Baxter Healthcare Corporation under the Trade Mark GEL-SYTE and from NDM Corporation of Dayton, Ohio under the name ClearSite. The material is disclosed in EP-A-0455324, and its clinical use is discussed by Evonne Fowler et al, Ostomy/Wound Management, The Journal for Extended Patient Care Management, Vol. 37, November/December 1991 at pages 39 to 45. The product is transparent and has the advantage that it adheres well to dry skin but is only weakly adherent to areas of moist skin, so that it can be removed from a wound site without damage to the area of healing. However, because of its multi-layer structure it is expensive to manufacture, and because it uses a dimensionally stable backing membrane, the extent to which the material can be stretched is limited, which is a disadvantage in some applications.

WO88/08310 discloses a sterile sealed flexible package containing a transparent sheet-form wound dressing comprising a wholly synthetic hydrophilic water-insoluble cross-linked polymer swollen with an aqueous liquid in which the polymer is capable of absorbing 0.9% by weight saline to form a gel containing at least 50% saline. In an example hydroxyethyl methacrylate (97.5 parts), hydroxyethyl acrylate (1 part), methacrylic acid (0.75 parts) and ethylene glycol dimethacrylate (0.5 parts) were mixed with a curing agent and the resulting polymerisation mixture was cast onto a flat surface and cured with UV light at 365 nm for 60 minutes. Since the polymer is for use in direct contact with the body, excess monomer which is cytotoxic has to be removed from the polymer by washing by immersion in water for a total of 24 hours followed by immersion in saline. Portions of the resulting sheet were then packed into polypropylene sheet containers and sterilised in an autoclave. The gel is stated to be strong enough to handle but, as a result of the prolonged washing treatment, it is not skin adhesive and a covering is applied over the wound dressing to maintain it in position.

WO98/19311 discloses cationic adhesive hydrogels useful as skin contact adhesives and which are compatible with aluminium and stainless steel electrodes. The hydrogels are made from cationic acrylates and in particular acrylic esters of quaternary chlorides. In an example, aqueous solutions of acryloyloxyethyl trimethyl ammonium chloride, potassium chloride and methylene bis-acrylamide, initiator in ethanol, and a buffer were mixed and photopolymerised at 50°C and at pH 4.2. The resulting gel is stated to have good adhesive and cohesive strength with little residue and to pass standards for biocompatibility and electrical properties developed by the Association for the Advancement of Medical Instrumentation (AMMI). However, the acid pH of the gel limits its applicability because it gives rise to haemolysis and cytotoxicity. The gel material looses cohesiveness and becomes brittle on absorption of water, and it is prone to distortion and loss of clarity on hydration and to give off drops of water.

### Adhesives for biomedical electrodes and sensors

WO95/31491 discloses polyacrylate gels cross-linked with long chain silyl methacrylates to form polymer adhesives for biomedical electrodes and sensors. Gel compositions based on acrylamide are disclosed but the only compositions disclosed as polymerising with photoinitiator are based on glycerol and not on water as solvent.

Medical electrodes for e.g. external cardiac pacing, transcutaneous electrical nerve stimulation (TENS) and neuromuscular stimulation are described in US-A-5205297. They include a protective cover layer, a conductive layer of carbon filled silicone rubber having a resistivity of about 100 Ω.cm and a self-supporting skin-contacting hydrogel layer of resistivity 1000 - 10000 Ω.cm. Natural or synthetic gums, polyacrylamides and polyacrylic acid and its salts may provide the basis for the hydrogel layer which, in the examples, contains both water and glycerin.

### Summary of the Invention

We have found that hydrogel or hydrogel-forming sheet material which is suitable for use as a wound dressing, and for other applications, can be made by photopolymerization in the presence of water. The resulting gel is clear, free of haemolytic unreacted monomer, and remains stretchable and cohesive on hydration. It is free of components which are liable to leach into a wound and hence disturb granulating tissues. It may be used without requiring washing to remove unreacted monomer, and can be produced in a continuous process. The mixture may also be photopolymerised in the substantial absence of water.

According to a first aspect of the invention, there is provided a composition which is photopolymerizable in the presence of water comprising:
(a) a first water-soluble monomer having a polymerisable olefinic group and a flexible hydrophilic chain and which is of formula: 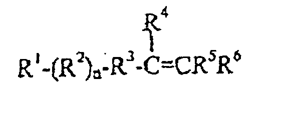 in which R¹ represents hydroxyl or C₁-C₄ alkoxy, R² represents C₂-C₃ alkoxy, R³ is -O- or -CO-, R⁴, R⁵ and R⁶ represent hydrogen or C₁-C₄ alkyl, and n is 5 - 10;
(b) a second water-soluble monomer having a polymerisable olefinic group and a group which imparts tackiness on curing, and which is a water soluble compound of the formula:

   CH₂=CR⁷R⁸

   wherein R⁷ represents hydrogen or methyl and R⁸ represents a non oligomeric polar or ionic organic group which imparts skin-adhesion but does not impart toxicity to the resulting polymer gel;
(c) a cross-linking agent;
(d) a photoinitiator and
(e) 2-20 wt% of a low molecular weight polyol.

In a further aspect the invention provides method for making a hydrogel composition which comprises exposing to light a composition as above. Embodiments of the gel can be used as a wound dressing or can be used as a conductive adhesive for medical electrodes.

### Description of Preferred Features

### First monomer

In the above composition, the first monomer is a water-soluble compound.which has olefinic unsaturation and a flexible hydrophillic chain, preferably an oligomeric polyoxyalkylene chain connected to an ethylene or other alkylene group. A preferred class of materials is of the formula: 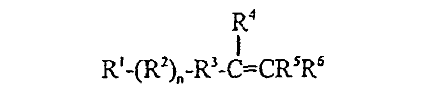 in which:
R¹ represents hydroxyl or C₁-C₄ alkoxy;
R² represents C₂-C₃ alkoxy;
R³ is -O- or -CO-;
R⁴, R⁵ and R⁶ represents hydrogen or C₁-C₄ alkyl; and
n is 1 - 25, typically about 5-10.

The polyoxyalkylene chain may be a polyethylene glycol chain that may contain minor amounts of polypropylene glycol or other units that do not interfere with its hydrophilic character or impart toxicity. In a particularly preferred class of compounds of the above formula, R¹ represents methoxy- or ethoxy-, R² represents ethoxy- (optionally with a minor amount of propoxy- or other alkoxy- units), R³ represents -CO-, R⁴ represents methyl and R⁵ and R⁶ represent hydrogen.

Preferred materials are of the formulae:

CH₃CH₂O(CH₂CH₂O)ₙCOC(CH₃)=CH₂

H₃CO(CH₂CH₂O)ₙCOC(CH₃)=CH₂ or

HO(CH₂CH₂O)ₙCOC(CH₃)=CH₂

in which n is as defined above. Also preferred for some applications are materials of the formula:

CH₂=CH(CH₃)-CO-[0CH₂CH₂(CH₃)]ₚ[0CH₂CH₂]_{q}0H

wherein p and q are positive integers, subject to the proviso that the sum of p and q is in the range 2-25, typically about 5-10.

All the above compounds should be in the form of water-soluble liquids. Their molecular weights may typically be in the range 200-700, preferably 300-600 and commonly about 400. For making wound dressing gels, the methoxy and ethoxy compounds are preferred. The methoxy compounds in particular have been found to give a more flexible and elastic gel than the ethoxy compounds. They are less expensive and are available in large as opposed to laboratory scale quantities. They have the further advantage compared to the corresponding ethoxy-compounds that lesser proportions may be needed to give a gel of the desired properties, which is an advantage because the above polyoxyalkylene monomers are relatively expensive. For making electrode adhesives, the hydroxy-compounds have also been found to give good results.

### Second monomer

The second monomer is a water soluble compound which may be of the formula:

CH₂=CR⁷R⁸

wherein R⁷ represents hydrogen or methyl and R⁸ represents a non-oligomeric polar or ionic organic group which imparts skin-adhesion but does not impart toxicity to the resulting polymer gel. For the production of flat sheet hydrogels for use in wound dressing and similar materials, R⁸ represents polar but non-ionic groups. Values of R⁸ may include

-CONR⁹R¹⁰

wherein R⁹R¹⁰ represent hydrogen, lower (C, - C₄) alkyl or lower hydroxyalkyl. Compounds of this class include acrylamide, methacrylamide, *N,N-*dimethylacrylamide and *N*-(2-hydroxypropyl)-methacrylamide. R⁸ may also include

-COOR¹¹

wherein R¹¹ represents a hydrogen atom or a C₁ - C₄ mono, di- or poly-hydroxyalkyl group. Compounds of this class include acrylic acid, methacrylic acid, 2-hydroxyethyl acrylate, 2-hydroxyethyl methacrylate, glyceryl monoacrylate or glyceryl monomethacrylate. R⁸ may also include substituted amino groups including cyclic groups to which the nitrogen atom is bonded, the cyclic group optionally being substituted with >C=O or an -OH group. A suitable compound is *N*-vinyl pyrrolidone. R⁸ may also include alkylsulfone groups, a suitable compound being vinyl methyl sulfone.

Where the intended product is an electro-conducting gel for use in electrodes for attachment to the body of human beings or animals, then the second monomer may provide conductivity-imparting, e.g. anionic groups, for example carboxylate or sulphonate groups which may be partly or wholly neutralised by a physiologically acceptable cation, for example sodium or potassium. Neutralisation can be important in applications where minimisation of haemotoxicity or cytotoxicity is desired. Thus the second monomer may be:

HO₃S-CH₂-C(CH₃)₂-HN-COCH=CH₂

which may be partly or completely neutralized with a physiologically acceptable cation, e.g. sodium or potassium.

### Cross-linking agent

The cross-linking agent is preferably of formula: 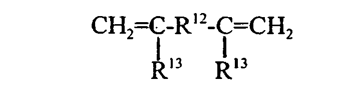 wherein R¹³ represents H or CH₃ and R¹² represents a polar linking group and is preferably water soluble. R¹² can be selected to provide the desired combination of properties and in general the greater the length between the olefinic groups, the more macroporous the gel is. Suitable cross-linking agents are water-soluble or water miscible diolefinic acrylates or methacrylates, e.g:

CH₂=CH(CH₃)-CO-O-CH₂-CH₂-O-COCH(CH₃)=CH₂

CH₂=CH-CO-NH-CH₂-NH-CO-CH=CH₂ and

CH₂=C(CH₃)-CO-O-(CH₂CH₂O)ₙ-O-CO-C(CH₃)=CH₂

wherein n is an integer.

When the preparation is carried out in an aqueous system, the cross linking agent is preferably water soluble.

### Further features of the composition

Where the first monomer is an ethoxy-terminated polyoxyethylene of the kind indicated above, the composition may comprise 0.5 - 5 parts by weight of the second monomer per part per weight of the first monomer. Where the first monomer is a methoxy-terminated material, there may be about 10 parts by weight of the second monomer per part by weight of the first monomer. The composition may also comprise 0.001 - 0.1 parts by weight of the cross linking agent per part by weight of the first monomer. The composition may further comprise water in an amount such that on curing it directly forms a hydrogel, and for example it may contain from 5 - 60 wt% of water, preferably in the case of sheet gels 5-40 wt%.

We have found that the inclusion of small amounts (e.g. 2-20 wt%) of a low molecular weight polyol (e.g. MW less than a 1000, preferably less than 500) may significantly increase the adhesiveness of the polymerised hydrogel. It is believed that part of the polyol becomes incorporated into the resulting polymer structure through chain transfer, and that part of the polyol may not become incorporated. Polyols that may be used include ethylene glycol, propylene glycol, low molecular weight polyethylene glycols and glycerol. The amount of polyol incorporated may be used to adjust the tackiness of the hydrogel composition to a desired value.

The uncured compositions of the invention may further comprise a thickener, especially in those instances where the weight of the first monomer is relatively low compared to that of the second monomer e.g. they are in a weight ratio of about1:10. For example, polyvinyl alcohol imparts viscosity to the uncured composition and permits the composition to be cast onto release paper. If it is not present, it becomes difficult to obtain a uniform layer. The amount incorporated is also selected having regard to the desired tackiness of the cured composition - an increase in the amount of polyvinyl alcohol incorporated generally brings about a reduction in the tackiness of the cured composition. Carbohydrate-based thickeners may also be incorporated, for example chitosan (which is preferred because it is believed to have healing properties), carrageenan or guar gum.

If the gel composition is not to be used extemporaneously, but has to be stored and/or transported before it is cured, addition of a free-radical polymerisation inhibitor, e.g. 4-methoxyphenol or hydroquinone, may be desirable to prevent premature polymerisation.

The composition may further comprise a biologically active material which is retained on curing and becomes gradually released from the cured composition when in position on the human being or animal body. In the case of a flat sheet hydrogel for use as a wound dressing agent, the biologically active material is a growth factor, antibacterial agent, anti-fungal agent, antiseptic agent, anaesthetic, debriding agent, anti-inflammatory agent, enzyme or nutrient. In the case of transdermal patches, the composition can further comprise a transdermally administrable drug.

### Curing the composition

The composition may be cured by UV light and may be pre-formed into a layer of thickness 0.1 - 3 mm, typically about 1.5 mm by pouring the mixture onto a substrate, and curing the mixture by light to form a water-insoluble sheet gel which is transparent, coherent, adhesive and water absorbent. The cured sheet is self-supporting and does not require internal reinforcement, which is an advantage where stretchability is a desideratum. However, if desired a reinforcing agent, for example a mesh of textile material, may be incorporated into the composition before it is cured. In the case of a sheet composition there may be also provided a backing sheet and a sheet of release paper to permit application to the skin. In the case of conductive compositions the substrate may be of metal sheet or foil e.g. of aluminium, tin or stainless steel, and the layer thickness may range up to about 1 mm.

Small quantities of the hydrogel can be made batchwise by passing polymerization mixtures into a tray, passing the tray past a UV light source and removing the resulting polymerized sheet from the tray. Larger quantities may be produced using an endless belt e.g. of released coated plastics material which passes successively through a casting station where polymerization mixture is poured onto the belt to a desired depth, a polymerization station where UV light is applied to the mixture, a stripping station where the sheet of hydrogel is removed from the belt, and a cutting station where the stripped sheet is cut to convenient size pieces.

The cut sheet may be sterilised e.g. by ethylene oxide gas or by gamma-irradiation which is preferred, and packed into a blister pack or in a sealed pouch.

### Uses of the composition

Particular applications of flat sheet gels are wound dressings e.g. for bums as explained above, for drug delivery patches and as microbiological swabs. The gel sheets may also be used in electrophoresis. The electro-conducting flat sheet may be used as a grounding plate, for ECG electrodes, as defibrillator pads, for external cardiac pacing electrodes, TENS electrodes and neuromuscular stimulation electrodes.

Embodiments of the invention are described in the following examples

### Example 1

### Water based gel using poly(ethylene glycol)ethyl ether methacrylate

An amber screw top bottle (100ml) was placed on an electronic balance and N,N-methylene-bis-acrylamide (0.025g; Sigma) was added, followed by de-ionised water (5g). The mixture was stirred vigorously until all of the N,N-methylene-bis-acrylamide had dissolved. There were then introduced into the bottle by means of pipette N,N-dimethylacrylamide (10g; Aldrich), poly(ethylene glycol)ethyl ether methacrylate (12g; Aldrich) and 2-hydroxy-2-methyl-1-phenyl-propan-1-one (0.5g, CIBA). Poly(ethylene glycol)ethyl ether methacrylate is a monomer of formula:

CH₃CH₂O(CH₂CH₂O)ₙCOCH(CH₃)=CH₂

The material used in this example was of molecular weight about 400.

The ingredients were mixed thoroughly and then poured into a polypropylene tray which was passed 10 times beneath UV curing apparatus comprising a Fusion F300S UV lamp connected to a Fusion P300m power supply and a Fusion LC6E conveyer running at a speed of 4 metres per minute. The bulb used was 15 cm long and had a wavelength λ of 200 - 400 nm. A self-supporting sheet of tacky gel was formed. Care was taken to ensure that the polymerization is complete and that unreacted monomer was not present. The gel was removed from the tray and placed on release paper. The gel was flexible and easy to remove from the siliconised surface of the release paper. It had the property of adhering well to dry skin but exhibiting little or no adhesion to moist skin. The gel could be incorporated into a siliconised blister pack or into a central region of a bandage having a peripheral region of conventional skin adhesive.

The gel maintained its transparency, stretchablilty and coherence on absorption of water. When a piece of the gel was immersed in water, its weight and thickness increased but it remained clear and handleable. It could be replaced on to dry skin to which it remained adherent. Bio-testing by extraction with saline and contact of the saline extract with red blood cells showed no evidence of haemolysis and cytotoxicity. It was concluded that the cured gel was free of toxic quantities of untreated monomer.

### Example 2

### Use of poly(ethyleneglycol) dimethacrylate as cross-linking agent

The procedure of Example 1 was repeated except that the N,N-methylene-bis-acrylamide was replaced by poly(ethyleneglycol) dimethacrylate (0.025g; Aldrich). The product was a self-supporting sheet of adhesive gel that adhered well to dry skin but not to moist skin.

### Example 3

### Polymerization of an anhydrous composition

The ingredients of Example 2 were mixed together to form a solution but with the de-ionised water omitted. The resulting solution cross-linked by UV light as before to give a self-supporting adhesive sheet that became swollen on contact with water. The cured composition may find utility where anhydrous highly water-absorbent adhesives for the human being or animal body are required.

### Example 4

### Polymerization of compositions containing relatively large amounts of water

The procedure of Example 2 was repeated except that 15g of de-ionised water were added. A self-supporting gel was produced. The experiment was repeated with more than 15g of water, and it was found that the solution become cloudy and there was phase separation, showing that for these monomers 15g of water represents a practical upper limit.

### Example 5

### Incorporation of a gauze reinforcement

The procedure of Example 2 was repeated except that a gauze support was placed in the polypropylene tray before the polymerizable monomers were added. After curing had taken place the gauze support became incorporated into the gel sheet. A polyurethane backing sheet was placed on one face of the gel sheet, and the other face was covered with a sheet of release paper to provide a self-supporting bandage.

### Example 6

### Conductive adhesive for an electrode using poly(ethyleneglycol)ethyl ether methacrylate

N,N-methylene-bis-acrylamide (0.4g, Sigma) was weighed into a 200ml beaker followed by 50g of de-ionised water. The mixture was stirred vigorously until all the N,N-methylene-bis-acrylamide had dissolved, after which 2-acrylamido-2-methyl-1-propane sulphonic acid (25g, Aldrich) were added. That material is a monomer of formula:

HO₃S-CH₂-C(CH₃)₂-HN-COCH=CH₂

The resulting solution was stirred until all traces of solid had disappeared, after which poly(ethyleneglycol)ethyl ether methacrylate (10g, Aldrich) and 0.1N sodium hydroxide (5g) were added. The resulting solution was poured into an amber screw top bottle and 2-hydroxy-2-methyl-1-phenyl propan-1-one (0.5g, CIBA, Daracure 1173) were added.

A piece of aluminium foil was placed in a polypropylene tray and some of the solution was transferred onto the foil by pipette to form a layer of depth about 1mm. The solution was covered with a piece of transparent release paper and the tray was passed through the Fusion UV curing system four times at a speed of 4m per minute. The release paper was removed and the gel was allowed to harden for about 10 minutes after which testing by means of a conductivity tester showed that the adhesive gel layer which had formed on the foil was electrically conductive. The release paper was then replaced.

### Example 7

### Increase in surface uniformity

The procedure of Example 6 was repeated except that 0.5g of polyvinyl alcohol was added at the same time as the methylene-bis-acrylamide. A conductive adhesive gel was formed which had a more uniform surface than that in the preceding example.

### Example 8

### Use of un-neutralised sulphonic acid containing monomer

The procedure of Example 6 was repeated except that the sodium hydroxide was omitted. A conductive adhesive gel was found to have formed on the aluminium foil.

### Example 9

### Use of neutralised sulphonic acid containing monomer

The procedure of Example 6 was repeated except that after addition of the poly(ethyleneglycol) ethyl ether methacrylate, the solution was treated with concentrated sodium hydroxide to adjust the pH into the range 6-7. Polymerisation as before gave a conductive adhesive gel with low toxicity to skin cells. Bio-testing using the procedure of Example 1 showed no evidence of haemolysis or cytotoxicity.

### Example 10

### Incorporation of a biologically active material

The procedure of Example 1 was repeated except that there was present 0.085g of dipyridamole which was detectable by eye because of its bright yellow color. On UV-curing the dipyridamole was found to have been incorporated into the adhesive composition from which it was gradually released over a period of 12 - 24 hours when the cured composition was placed in water.

### Example 11

### Use of propylene glycol to increase tackiness

2-Acrylamido-2-methyl-1-propanseulphonic acid (12.99g) was mixed with de-ionised water (20ml) and solid sodium hydroxide (3.11g) was added followed by poly(ethylene glycol) ether methacrylate (2g). The resulting mixture was stirred. A separate mixture of N,N-meihylene-bis-acrylamide (0.175g) in de-ionised water (5g) was stirred for two minutes, after which curing agent (Darocure 1173, 0.25g) and propylene glycol (varying amounts from 1 - 5g) were added. The resulting polymerisable mixture was coated onto glass sheet and cured by passage twice through the Fusion UV curing apparatus of Example 1 to give a transparent hydrogel sheet. All samples where propylene glycol had been added exhibited greater tackiness than for an equivalent mixture containing no propylene glycol. As the amount of propylene glycol was increased from I to 3g the tackiness increased. At values of 4 - 5g, the tackiness was undesirably high and when release paper.was placed over the sheet, the hydrogel became stuck to the release paper.

### Example 12

### Water-based skin-adhesive gel using methoxypolyethylene glycol methacrylate

A glass beaker (2500ml) was placed on an electronic balance and polyvinyl alcohol (0.09g; BDH) was added, followed by de-ionised water (5g). The mixture was stirred vigorously until all of the polyvinyl alcohol had dissolved. There were then introduced into the beaker by means of pipette N,N-dimethylacrylamide (10g; Aldrich), methoxypoly-ethylene glycol methacrylate (1g; Inspec), polyethylene glycol dimethacrylate (0.15g; Aldrich), 2-hydroxy-2-methyl-1-phenyl-propan-1-one (0.4g; CIBA) and propane 1,2-diol (1g; Aldrich). Methoxypolyethylene glycol methacrylate is a compound of chemical formula:

H₃C-O-(CH₂CH₂O)ₙCO-C(CH₃)=CH₂

The material used in this example was of molecular weight about 400.

The ingredients were mixed thoroughly and then formed into a thin layer on a piece of siliconised release paper. This was passed 10 times beneath the Fusion UV curing apparatus of Example 1 running under the conditions there stated. A self-supporting sheet of tacky gel was formed on the release paper. The gel was flexible and easy to remove from the siliconised release paper. It had the property of adhering well to dry skin but exhibiting little or no adhesion to moist skin. The gel could be incorporated into a siliconised blister pack or into a central region of a bandage having a peripheral region of conventional skin adhesive.

The gel maintained its transparency, stretchablilty and coherence on absorption of water. Its weight, area and thickness increased, but it remained clear and handleable. It could be replaced on to dry skin to which it remained adherent. Bio-testing by extraction with saline and contact of the saline extract with red blood cells showed no evidence of haemolysis and cytotoxicity. It was concluded that the cured gel was free of toxic quantities of untreated monomer.

Further evaluation of the gel was carried out, and in particular it passed tests for cytotoxicity, skin irritation and haemolysis at SafePharm Laboratories, Derby, UK.

The gel was tested for skin adhesion using the following procedure. A layer of the gel covered on both faces with siliconised release paper was cut to form a sample 2.5cm wide and 10cm long. At one end of the sample, a small area of the gel layer was separated from the release paper and a bulldog clip was attached to the gel layer. Release paper was removed from one face, which was applied to shaven human skin, the release paper on the other face being left in place to avoid the gel drying out. Care was taken to eliminate air bubbles, and a 500g weight was passed 5-6 times over the release paper to press the underlying gel layer firmly against the skin. The bulldog clip was connected to one end of a force measuring device whose other end was connected to an electric motor via a thread. Energising the motor caused it to tension the thread and pull the gel layer from the skin. The force to pull the gel layer off the skin was recorded as about 1 Newton.

The gel was also tested for water uptake. A weighed sample of the gel was placed in a solution of sodium chloride (5.6g) in water (600ml), and was removed and re-weighed at timed intervals. After 24 hours it was found to have taken up about 93% of its own weight of water.

### Example 13

### Effect of omission of PVA

Example 12 was repeated except that the PVA was omitted. A self-supporting gel was produced, but it showed less uniformity, flexibility and adhesion to dry skin than the gel of Example 12.

### Example 14

### Variation in the amount ofpropylene glycol

Example 12 was repeated using quantities of propane-1,2-diol in the range 1-3g. The resulting gels exhibited an increase in skin adhesion with increasing propane- 1,2-diol content, but at 3g phase separation occurred which is undesirable.

### Example 15

### Evaluation of alternatives to methoxypolyethylene glycol methacrylate

Example 12 was repeated except that the above monomer was replaced by polyalkylene glycol methacrylate (1g; Inspec). The polyalkylene glycol methacrylate : used was a modified oligomer of formula:

CH₂=CH(CH₃)-CO-(0CH₂CH₂(CH₃)]₆[OCH₂CH₂]₃OH

The resulting product was a self-supporting sheet of gel, but it did not adhere well to wet, moist or dry skin. The gel was relatively brittle and lost flexibility when exposed to water: Its water uptake was less than that of the gel of Example 12.

Example 12 was also repeated using polyethylene glycol monomethacrylate (1g; Inspec) instead of the above monomer. The material used was of formula:

HO-(CH₂CH₂O)ₙ-CO-C(CH₃)=CH₂

and had a molecular weight of about 400. The resulting gel was similar to that reported above. Its water uptake was surprisingly poor compared to the gel of Example 12.

### Example 16

### Conductive adhesive for electrodes using polyethylene glycol monomethacrylate

Sodium hydroxide pellets (2.51 g; BDH) were mixed with de-ionised water (8g) in a 250 ml glass beaker, and the mixture was allowed to cool to room temperature, after which 2-acrylamido-2-methyl-1-propanesulphonic acid (13g; Lubrazol) was gradually added. The resulting solution was stirred and allowed to cool to room temperature, after which there were added polyethylene glycol monomethacrylate (1g, Inspec), polyethylene glycol dimethacrylate (1.5g; Aldrich), 2-hydroxy-2-methyl-1-phenyl propan-1-one (0.25g; CIBA) and a mixture of 4-methoxyphenol (0.01g; Aldrich) and propan-1,2-diol (9g; Aldrich). It is believed that the satisfactory results obtained using polyethylene glycol monomethacrylate in this example may be because of the different co-monomers used and also because of the different properties required in an electrode adhesive, where brittleness of the resulting gel is less of a problem.

Borders were formed along a piece of aluminium foil, and a layer of the above solution 1 mm deep was applied to it from a pipette. The resulting solution was passed 4-5 times beneath the Fusion UV curing system of Fig. I running under the conditions therein stated. The resulting cured gel was found to be electrically conductive. It was covered with a sheet of release paper.

The gel passed the same bio-evaluation tests as the gel of Example 12 and when tested for skin adhesion as described in that example required a force of 1.5-2 Newtons for its removal. The skin adhesion was higher than the gel of example 12 because in this field of use it is important to maximise contact between skin and electrode.

### Example 17

### Variation in amount of propane 1,2-diol

The procedure of example 16 was repeated with quantities of propane-1,2-diol in the range 8-12g. The resulting cured gels all showed an increase in adhesion with incerase in propane-1,2-diol content, but after 10g, a phase separation occurred which is undesirable.

### Example 18

### Use of 2-hydroxyethyl acrylate as a monomer

The procedure of example 12 was repeated except that instead of N,N-dimethylacrylamide there was used 2-hydroxyethyl acrylate (8g; Fluka). The resulting product was a self-supporting gel that adhered well to the skin.

### Example 19

### Use of glyceryl methacrylate (GMA) as a monomer

The procedure of example 12 was repeated except that instead of N,N-dimethylacrylamide there was used GMA (8g). The resulting product was a self-supporting gel that adhered well to the skin.

### Example 20

### Use of a carbohydrate to impart viscosity to the uncured composition

The procedure of Example 12 was repeated except that the PVA was replaced by 0.2% chitosan (Vanson) in 1% acetic acid. The resulting product was a self supporting gel that adhered well to the skin.

## Claims

1. A composition which is photopolymerizable in the presence of water comprising:
(a) a first water-soluble monomer having a polymerisable olefinic group and a flexible hydrophilic chain and which is of formula; 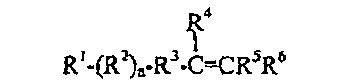 in which R¹ represents hydroxyl or C₁-C₄ alkoxy, R² represents C₂-C₃ alkoxy, R³ is -O- or -CO-, R⁴, R⁵ and R⁶ represent hydrogen or C₁-C₄ alkyl, and n is 5 - 10;
(b) a second water-soluble monomer having a polymerisable olefinic group and a group which imparts tackiness on curing, and which is a water soluble compound of the formula:
CH₂=CR⁷R⁸
wherein R⁷ represents hydrogen or methyl and R⁸ represents a non oligomeric polar or ionic organic group which imparts skin-adhesion but does not impart toxicity to the resulting polymer gel;
(c) a cross-linking agent;
(d) a photoinitiator; and
(e) 2-20 wt% of a low molecular weight polyol.

2. The composition of claim 1, wherein the first monomer is of the formula defined in claim 1, in which R¹ represents methoxy- or ethoxy-, R² represents ethoxy- or a mixture of ethoxy- and propoxy-, R³ is -CO-, R⁴ represents hydrogen or methyl and R⁵ and R⁶ represent hydrogen.

3. The composition of claim 1, wherein the first monomeris of formula:
CH₃CH₂O(CH₂CH₂O)ₙCOC(CH₃)=CH₂
wherein n is 5-10.

4. The composition of claim 1, wherein the first monomer is of formula:
CH₃O(CH₂CH₂O)ₙCOC(CH₃)-CH₂
wherein n is 5-10.

5. The composition of claim 1, wherein the first monomer is of the formula:
HO(CH₂CH₂O)ₙCOC(CH₃)=CH₂
wherein n is 5-10.

6. The composition of claim 1, wherein the first monomer is of formula:
CH₂=CH(CH₃)-CO-[OCH₂CH₂(CH₃)]ₚ[OCH₂CH₂)_{q}OH
wherein p and q arc positive integers, subject to the proviso that the sum of p and q is in the range 5-10.

7. The composition of any preceding claim, wherein the average molecular weight of the first monomer is about 200-700.

8. The composition of any preceding claim, wherein the average molecular weight of the first monomer is about 300-600.

9. The composition of any preceding claim, wherein R⁸ is
-CONR⁹R¹⁰
wherein R⁹R¹⁰ represent hydrogen, lower (C₁ - C₄) alkyl or lower hydroxyalkyl.

10. The composition of any of claims 1-8, wherein the second monomer is acrylamide, methacrylamide, *N,N*-dimethylacrylamide and *N-*(2-hydroxypropyl)-methacrylamide.

11. The composition of any of claims 1-8, wherein R⁸ is
-COOR¹¹
wherein R¹¹ represents a hydrogen atom or a C₁-C₄ mono, di- or poly-hydroxyalkyl group.

12. The composition of any of claims 1-8, wherein the second monomer is acrylic acid, methacrylic acid, 2-hydroxyethyl acrylate, 2-hydroxyethylmethacrylate, glyceryl monoacrylate or glyceryl monomethacrylate.

13. The composition of any of claims 1-8, wherein R⁸ is an alkyl substituted amino groups or a cyclic group containing a. nitrogen atom to which the vinyl group is bonded, the cyclic group optionally containing a >C=O or-OH group.

14. The composition of any of claims 1-8, wherein the second monomer is N-vinyl pyrrolidone.

15. The composition of any of claims 1-8, wherein R⁸ is an alkylsulfone group.

16. The composition of any of claims 1-8, wherein the second monomer provides an anionic group.

17. The composition of claim 16, wherein the anionic groups is carboxylate or sulfonate which may be partly or completely neutralised by cations of sodium or potassium.

18. The composition of claim 17, wherein the second monomer is:
HO₃S-CH₂-C(CH₃)₂-HN-COCH=CH₂
which may be partly or completely neutralised by cations of sodium or potassium.

19. The composition of any preceding claim wherein the cross-linking agent is of formula: 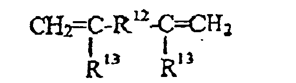 wherein R¹³ represents H or CH₃ and R¹² represents a polar linking group.

20. The composition of claim 19, wherein the cross-linking agent is:
CH₂=CH-CO-NH-CH₂-NH-CO-CH=CH₂ or
CH₂=C(CH₃)-CO-O-(CH₂CH₂O)ₙ-O-CO-C(CH₃)-CH₂
wherein n is an integer.

21. The composition of any preceding claim, wherein there are present 0.5 - 10 or more parts by weight of the second monomer per part by weight or the first monomer.

22. The composition of claim 21, wherein there arc present 0.001 to 0.1 parts by weight of cross-linking agent per part by weight of the first monomer.

23. The composition of any preceding claim, further comprising water in an amount such that the composition on curing is a hydrogel.

24. The composition of any preceding claim, comprising 5-40 wt% water.

25. The composition of any preceding claim, wherein the low molecular weight polyol is selected from ethylene glycol, propylene glycol, low molecular weight polyethylene glycols, low molecular weight polyethylene/polypropylene glycols and glycerol.

26. The composition of any preceding claim, further comprising a biologically active material.

27. The composition of claim 26, wherein the biologically active material is a growth factor, antibacterial agent, anti-fungal agent, antiseptic agent, anaesthetic, debriding agent, anti-inflammatory agent, enzyme or nutrient.

28. The composition claims 26 or 27, further comprising a transdermally administrable drug.

29. An adhesive composition obtainable by curing a composition as defined in any of claims 1 to 28.

30. The composition of claim 29, which is in the form of a film or sheet.

31. The composition of claim 30 which is of thickness 0.1 -3mm.

32. The composition of claim 30 or claim 31, which is sterile.

33. A wound dressing comprising an adhesive composition as claimed in any of claims 29 to 32.

34. An electrode for attachment to the body of a human or animal comprising an adhesive composition as claimed in any of claims 29 to 32.

35. A transdermal patch comprising an adhesive composition as claimed in any of claims 29 to 32.

36. A method of making a hydrogel composition which comprises exposing to light a composition as claimed in any of claims 1 to 28.

37. The method of claim 36, wherein the composition is formed into a thin layer before it is cured, whereby the product is a Him.

38. The method of claim 36 or claim 37, wherein the composition is packed into a container without a prior washing step.

39. The method of claim 36, 37 or 38, wherein the composition is sterilised by exposure to γ-radiation.

## Patentansprüche

1. Zusammensetzung, die in Gegenwart von Wasser photopolymerisierbar ist, enthaltend:
(a) ein erstes wasserlösliches Monomer mit einer polymerisierbaren olefinischen Gruppe und einer flexiblen hydrophilen Kette und das folgende Formel hat: 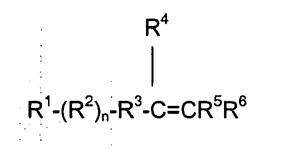 wobei R¹ Hydroxyl oder C₁-C₄ Alkoxy, R² C₂-C₃ Alkoxy, R³ -O- oder -CO-, R⁴, R⁵ und R⁶ Wasserstoff oder C₁-C₄ Alkyl und n 5 - 10 ist;
(b) ein zweites wasserlösliches Monomer mit einer polymerisierbaren olefinischen Gruppe und einer Gruppe, die nach Härtung Klebkraft verleiht, und das eine wasserlösliche Verbindung der folgenden Formel ist:
CH₂=CR⁷R⁸
wobei R⁷ Wasserstoff oder Methyl und R⁸ eine nicht oligomere polare oder ionische organische Gruppe ist, die Haftung an der Haut verleiht aber das resultierende Polymergel nicht toxisch macht;
(c) ein Vernetzungsmittel;
(d) einen Photoinitiator; und
(e) 2-20 Gewichtsprozent eines Polyols mit niedrigem Molekulargewicht.

2. Zusammensetzung nach Anspruch 1, wobei das erste Monomer die in Anspruch 1 definierte Formel hat, wobei R¹ Methoxy- oder Ethoxy-, R² Ethoxy- oder eine Mischung von Ethoxy und Propoxy, R²-CO-, R⁴ Wasserstoff oder Methyl und R⁵ und R⁶ Wasserstoff ist.

3. Zusammensetzung nach Anspruch 1, wobei das erste Monomer die folgende Formel hat:
CH₃CH₂O(CH₂CH₂O)ₙCOC(CH₃)=CH₂
wobei n 5-10 ist.

4. Zusammensetzung nach Anspruch 1, wobei das erste Monomer die folgende Formel hat:
CH₃O(CH₂CH₂O)ₙCOC(CH₃)=CH₂
wobei n 5-10 ist.

5. Zusammensetzung nach Anspruch 1, wobei das erste Monomer die folgende Formel hat:
HO(CH₂CH₂O)ₙCOC(CH₃)=CH₂
wobei n 5-10 ist.

6. Zusammensetzung nach Anspruch 1, wobei das erste Monomer die folgende Formel hat:
CH₂=CH(CH₃)-CO-[OCH₂CH₂(CH₃)]_{P}[OCH₂CH₂]_{q}OH
wobei p und q positive ganze Zahlen sind mit der Maßgabe, dass die Summe von p und q im Bereich von 5 bis 10 liegt.

7. Zusammensetzung nach einem der vorhergehenden Ansprüche, wobei das mittlere Molekulargewicht des ersten Monomers etwa 200-700 ist.

8. Zusammensetzung nach einem der vorhergehenden Ansprüche, wobei das mittlere Molekulargewicht des ersten Monomers etwa 300-600 ist.

9. Zusammensetzung nach einem der vorhergehenden Ansprüche, wobei R⁸
-CONR⁹R¹⁰
ist, wobei R⁹ R¹⁰ Wasserstoff, eine niedrige (C₁-C₄) Alkyl oder niedriges Hydroxyalkyl ist.

10. Zusammensetzung nach einem der Ansprüche 1-8, wobei das zweite Monomer Acrylamid, Methacrylamid, N,N-Dimethylacrylamid und N-(2-Hydroxypropyl)-methacrylamid ist.

11. Die Zusammensetzung nach einem der Ansprüche 1-8, wobei R⁸
-COOR¹¹
ist, wobei R¹¹ Wasserstoff oder eine C₁-C₄ Mono-, Di- oder Polyhydroxyalkylgruppe ist.

12. Zusammensetzung nach einem der Ansprüche 1-8, wobei das zweite Monomer Acrylsäure, Methacrylsäure, 2-Hydroxyethylacrylat, 2-Hydroxyethylmethacrylat, Glycerylmonoacrylat oder Glycerylmonomethacrylat ist.

13. Zusammensetzung nach einem der Ansprüche 1-8, wobei R⁸ eine alkylsubstituirerte Aminogruppe oder eine zyklische Gruppe enthaltend ein Stickstoffatom, an das die Vinylgruppe gebunden ist, wobei die zyklische Gruppe gegebenenfalls eine >C=O oder -OH Gruppe enthält.

14. Zusammensetzung nach einem der Ansprüche 1-8, wobei das zweite Monomer N-Vinylpyrrolidon ist.

15. Zusammensetzung nach einem der Ansprüche 1-8, wobei R⁸ eine Alkylsulfongruppe ist.

16. Zusammensetzung nach einem der Ansprüche 1-8, wobei das zweite Monomer eine anionische Gruppe liefert.

17. Zusammensetzung nach Anspruch 16, wobei die anionische Gruppe Carboxylat oder Sulfonat ist, das ganz oder teilweise durch Natrium- oder Kaliumkationen neutralisiert sein kann.

18. Zusammensetzung nach Anspruch 17, wobei das zweite Monomer
HO₃S-CH₂-C(CH₃)₂-HN-COCH=CH₂
ist, das ganz oder teilweise durch Natrium- oder Kaliumkationen neutralisiert sein kann.

19. Zusammensetzung nach einem der vorhergehenden Ansprüche, wobei das Vernetzungsmittel die folgende Formel hat: 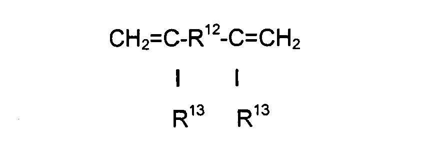 wobei R¹³ H oder CH₃ und R¹² eine polare Verknüpfungsgruppe ist.

20. Zusammensetzung nach Anspruch 19, wobei das Vernetzungsmittel:
CH₂=CH-CO-NH-CH₂-NH-CO-CH=CH₂
oder
CH₂=C(CH₃)-CO-O-(CH₂CH₂O)ₙ-O-CO-C(CH₃)=CH₂
ist, wobei n eine ganze Zahl ist.

21. Zusammensetzung nach einem der vorhergehenden Ansprüche, wobei pro Gewichtsteil an erstem Monomer 0,5 -10 oder mehr Gewichtsteile zweites Monomer vorliegen.

22. Zusammensetzung nach Anspruch 21, wobei pro Gewichtsteil an ersten Monomer 0,001 bis 0,1 Gewichtsteile Vernetzungsmittel vorliegen.

23. Zusammensetzung nach einem der vorhergehenden Ansprüche, zudem enthaltend Wasser in einer Menge, die ausreicht, dass die Zusammensetzung nach Härtung ein Hydrogel ist.

24. Zusammensetzung nach einem der vorhergehenden Ansprüche enthaltend 5 bis 40 Gewichtsprozent Wasser.

25. Zusammensetzung nach einem der vorhergehenden Ansprüche, wobei das Polyol mit niedrigem Molekulargewicht ausgewählt ist unter Ethylenglycol, Propylenglycol, Polyethylenglycolen mit niedrigem Molekulargewicht, Polyethylen/Polypropylenglycolen mit niedrigem Molekulargewicht und Glycerin.

26. Zusammensetzung nach einem der vorhergehenden Ansprüche, zudem enthaltend ein biologisch aktives Material.

27. Zusammensetzung nach Anspruch 26, wobei das biologisch aktive Material ein Wachstumsfaktor, antibakterielles Mittel, Antipilzmittel, Antiseptisches Mittel, Narkosemittel, Dibridementmittel, entzündungshemmendes Mittel, Enzym oder Nährstoff ist.

28. Zusammensetzung nach Anspruch 26 oder 27, zudem enthaltend eine transdermal verabreichbare Arznei.

29. Eine haftende Zusammensetzung erhältlich durch Härtung einer Zusammensetzung nach einem der Ansprüche 1 bis 28.

30. Zusammensetzung nach Anspruch 29 in Form einer Folie oder eines Blattes.

31. Zusammensetzung nach Anspruch 30 mit einer Dicke von 0,1 bis 3 mm.

32. Zusammensetzung nach Anspruch 30 oder Anspruch 31, die steril ist.

33. Wundverband enthaltend eine haftende Zusammensetzung nach einem der Ansprüche 29 bis 32.

34. Elektrode zum Anbringen an dem Körper eines Menschen oder eines Tieres enthaltend eine haftende Zusammensetzung nach einem der Ansprüche 29 bis 32.

35. Transdermales Pflaster enthaltend eine haftende Zusammensetzung nach einem der Ansprüche 29 bis 32.

36. Verfahren zur Herstellung einer Hydrogelzusammensetzung, das das Aussetzen einer Zusammensetzung nach einem der Ansprüche 1 bis 28 an Licht umfasst.

37. Verfahren nach Anspruch 36, wobei die Zusammensetzung vor der Härtung zu einer dünnen Schicht geformt wird, sodass das Produkt eine Folie ist.

38. Verfahren nach Anspruch 36 oder 37, wobei die Zusammensetzung ohne einem vorhergehenden Waschschritt in einen Behälter gepackt wird.

39. Verfahren nach Anspruch 36, 37 oder 38, wobei die Zusammensetzung durch γ-Bestrahlung sterilisiert wird.

## Revendications

1. Composition photopolymérisable en présence d'eau, comprenant :
(a) un premier monomère soluble dans l'eau ayant un groupe oléfinique polymérisable et une chaîne hydrophile flexible, et qui a pour formule : 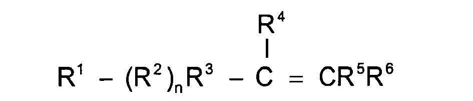 où R¹ est un hydroxyle ou alcoxy en C₁ à C₄, R² est un alcoxy à C₂ ou C₃, R³ est - O- ou -CO-, R⁴, R⁵ et R⁶ représentent l'hydrogène ou un alkyle en C₁ à C₄, et n est compris entre 5 et 10 ;
(b) un deuxième monomère soluble dans l'eau ayant un groupe oléfinique polymérisable et un groupe qui confère une adhésivité lors du durcissement, et qui est un composé soluble dans l'eau ayant pour formule :
CH₂=CR⁷R⁸
où R⁷ représente l'hydrogène ou le méthyle et R⁸ représente un groupe polaire non oligomère ou un groupe organique ionique qui confère une adhérence cutanée et non une toxicité au gel polymère résultant;
(c) un agent de réticulation;
(d) un photo-initiateur ; et
(e) 2 à 20 % en poids d'un polyol de faible masse moléculaire.

2. Composition selon la revendication 1, dans laquelle le premier monomère a la formule définie dans la revendication 1, dans laquelle R¹ est un méthoxy- ou éthoxy-, R² est un éthoxy- ou un mélange d'éthoxy- et de propoxy-, R³ est -CO-, R⁴ représente l'hydrogène ou le méthyle et R⁵ et R⁶ représentent l'hydrogène.

3. Composition selon la revendication 1, dans laquelle le premier monomère a pour formule :
CH₃CH₂O(CH₂CH₂O)ₙCOC(CH₃)=CH₂
où n est compris entre 5 et 10.

4. Composition selon la revendication 1, dans laquelle le premier monomère a pour formule :
CH₃O(CH₂CH₂O)ₙCOC(CH₃)=CH₂
où n est compris entre 5 et 10.

5. Composition selon la revendication 1, dans laquelle le premier monomère a pour formule :
HO(CH₂CH₂O)ₙCOC(CH₃)=CH₂
où n est compris entre 5 et 10.

6. Composition selon la revendication 1, dans laquelle le premier monomère a pour formule :
CH₂=CH(CH₃)-CO-[OCH₂CH₂(CH₃)]ₚ[OCH₂CH₂]_{q}OH
où p et q sont des nombres entiers positifs, soumis à la condition que la somme de p et q est comprise dans la plage de 5 à 10.

7. Composition selon l'une quelconque des revendications précédentes, dans laquelle la masse moléculaire moyenne du premier monomère est d'environ 200 à 700.

8. Composition selon l'une quelconque des revendications précédentes, dans laquelle la masse moléculaire moyenne du premier monomère est d'environ 300 à 600.

9. Composition selon l'une quelconque des revendications précédentes, dans laquelle R⁸ est
-CONR⁹R¹⁰
où R⁹R¹⁰ représente l'hydrogène, un alkyle en C₁ à C₄ inférieur ou un hydroxyalkyle inférieur.

10. Composition selon l'une quelconque des revendications 1 à 8, dans laquelle le deuxième monomère est l'acrylamide, le méthacrylamide, le N,N-diméthylacrylamide et le N-(2-hydroxypropyl)-méthacrylamide.

11. Composition selon l'une quelconque des revendications 1 à 8, dans laquelle R⁸ est
-COOR¹¹
où R¹¹ représente un atome d'hydrogène ou un groupe mono, di- ou polyhydroxyalkyle en C₁ à C₄.

12. Composition selon l'une quelconque des revendications 1 à 8, dans laquelle le deuxième monomère est l'acide acrylique, l'acide méthacrylique, l'acrylate de 2-hydroxyéthyle, le 2-hydroxyéthylméthacrylate, le monoacrylate de glycéryle ou le monométhacrylate de glycéryle.

13. Composition selon l'une quelconque des revendications 1 à 8, dans laquelle R⁸ est un groupe amino substitué par un alkyle ou un groupe cyclique contenant un atome d'azote auquel est lié le groupe vinyle , le groupe cyclique contenant éventuellement un groupe a > C-O ou -OH.

14. Composition selon l'une quelconque des revendications 1 à 8, dans laquelle le deuxième monomère est la N-vinylpyrrolidone.

15. Composition selon l'une quelconque des revendications 1 à 8, dans laquelle R⁸ est un groupe alkylsulfone.

16. Composition selon l'une quelconque des revendications 1 à 8, dans laquelle le deuxième monomère fournit un groupe anionique.

17. Composition selon la revendication 16, dans laquelle les groupes anioniques sont le carboxylate ou le sulfonate, qui peut être neutralisé partiellement ou complètement grâce à des cations de sodium ou de potassium.

18. Composition selon la revendication 17, dans laquelle le deuxième monomère est :
HO₃S-CH₂-C(CH₃)₂-HN-COCH=CH₂
qui peut être neutralisé partiellement ou complètement grâce à des cations de sodium ou de potassium.

19. Composition selon l'une quelconque des revendications précédentes,
dans laquelle l'agent de réticulation a pour formule : 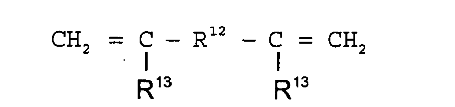 où R¹³ représente H ou CH₃ et R¹² représente un groupe de liaison polaire.

20. Composition selon la revendication 19, dans laquelle l'agent de réticulation est :
CH₂=CH-CO-NH-CH₂-NH-CO-CH=CH₂ ou
CH₂=C(CH₃)-CO-O-(CH₂CH₂O)ₙ-O-CO-C(CH₃)=CH₂
où n est un entier.

21. Composition selon l'une quelconque des revendications précédentes, dans laquelle 0,5 à 10 de parties en poids ou plus du deuxième monomère sont présentes par partie en poids du premier monomère.

22. Composition selon la revendication 21, dans laquelle 0,001 à 0,1 partie en poids de l'agent de réticulation sont présentes par partie en poids du premier monomère.

23. Composition selon l'une quelconque des revendications précédentes, comprenant en outre l'eau en une quantité telle que la composition lors du durcissement est un hydrogel.

24. Composition selon l'une quelconque des revendications précédentes, comprenant de 5 à 40 % en poids d'eau.

25. Composition selon l'une quelconque des revendications précédentes, dans laquelle le polyol de faible masse moléculaire est choisi parmi l'éthylène glycol, le propylène glycol, les polyéthylène glycols de faible masse moléculaire, les polyéthylène/polypropylène glycols et glycérol de faible masse moléculaire.

26. Composition selon l'une quelconque des revendications précédentes, comprenant en outre une matière active d'un point de vue biologique.

27. Composition selon la revendication 26, dans laquelle la matière active d'un point de vue biologique est un facteur de croissance, un agent antibactérien, un agent antifongique, un agent antiseptique, un anesthésiant, un agent débridant, un agent anti-inflammatoire, une enzyme ou un nutriment.

28. Composition selon la revendication 26 ou 27, comprenant en outre un médicament administrable par voie transdermique.

29. Composition adhésive pouvant être obtenue en durcissant une composition selon l'une quelconque des revendications 1 à 28.

30. Composition selon la revendication 29, qui se présente sous la forme d'un film ou d'une feuille.

31. Composition selon la revendication 30, qui a une épaisseur de 0,1 à 3 mm.

32. Composition selon la revendication 30 ou la revendication 31, qui est stérile.

33. Pansement comprenant une composition adhésive selon l'une quelconque des revendications 29 à 32.

34. Electrode destinée à être fixée au corps d'un être humain ou d'un animal comprenant une composition adhésive selon l'une quelconque des revendications 29 à 32.

35. Patch transdermique comprenant une composition adhésive selon l'une quelconque des revendications 29 à 32.

36. Procédé de production d'une composition d'hydrogel comprenant l'exposition à la lumière d'une composition selon l'une quelconque des revendications 1 à 28.

37. Procédé selon la revendication 36, dans lequel la composition est transformée en une couche mince avant d'être durcie, moyennant quoi le produit est un film.

38. Procédé selon la revendication 36 ou 37, dans lequel la composition est emballée dans un conteneur sans étape de lavage préalable.

39. Procédé selon la revendication 36, 37 ou 38, dans lequel la composition est stérilisée par l'exposition au rayonnement y.
